# FASCICULE DE BREVET EUROPEEN

(11) **EP 2 590 662 B1**
(45) Date de publication et mention de la délivrance du brevet: **20.04.2016**
(21) Numéro de dépôt: 11743093.4
(22) Date de dépôt: 05.07.2011
(51) Int. Cl.: A23L 33/10, A23L 33/17, A61K 35/74, C07K 14/00, A61P 29/00, C12N 1/20, A61K 38/16, C12P 19/04, C12P 1/04

(54) **COMPLEXE MACROMOLECULAIRE PROVENANT DE BIFIDOBACTERIUM LONGUM ET UTILISATION DUDIT COMPLEXE POUR PREVENIR ET TRAITER LES RHUMATISMES INFLAMMATOIRES**
MAKROMOLEKULARER KOMPLEX AUS BIFIDOBACTERIUM LONGUM UND VERWENDUNG DIESES KOMPLEXES FÜR VORBEUGUNG UND BEHANDLUNG VON ENTZÜNDLICHEM RHEUMATISMUS
MACROMOLECULAR COMPLEX FROM BIFIDOBACTERIUM LONGUM AND USE OF SAID COMPLEX FOR PREVENTING AND TREATING INFLAMMATORY RHEUMATISM

(30) Priorité: 05.07.2010 FR 1055432
(43) Date de publication de la demande: 15.05.2013
(73) Titulaire: Bifinove, 59000 Lille (FR)
(72) Inventeur: BLAREAU, Jean-Pierre, F-59114 Steenvoorde (FR); COLAVIZZA, Michel, F-59172 Roeulx (FR); HUGUET, Frédéric, F-59000 Lille (FR); ROMOND, Charles, F-59110 La Madeleine (FR)
(74) Mandataire: Cabinet Plasseraud
(86) Numéro de dépôt international: PCT/FR2011/051593
(87) Numéro de publication internationale: WO 2012/004522

(56) Documents cités:
- WO-A2-2006/040485
- DATABASE BIOSIS [Online] BIOSCIENCES INFORMATION SERVICE, PHILADELPHIA, PA, US; avril 2007 (2007-04), SHEIL BARBARA ET AL: "Modulation of the commensal flora by probiotic feeding delays the onset and decreases the severity of disease in the collagen-induced arthritis (CIA) model in a strain-dependent manner", XP002620173, Database accession no. PREV200700603452
- SUZUKI T. ET AL.: "Inhibition of bacterial translocation from the gastrointestinal tract of mice by oral administration of a culture condensate of Bifidobacterium longum.", J. VET. MED. SCI., vol. 59, no. 8, 1997, pages 665-669, XP002620174,
- MENARD S ET AL: "LACTIC ACID BACTERIA SECRETE METABOLITES RETAINING ANTI-INFLAMMATORY PROPERTIES AFTER INTESTINAL TRANSPORT", GUT, BRITISH MEDICAL ASSOCIATION, LONDON, UK, vol. 53, no. 6, 1 juin 2004 (2004-06-01), pages 821-828, XP008046639, ISSN: 0017-5749, DOI: DOI:10.1136/GUT.2003.026252
- SANCHEZ B. ET AL.: "Exported proteins in probiotic bacteria : adhesion to intestinal surfaces, host immunomodulation and molecular cross-talking with the host.", FEMS IMMUNOL. MED. MICROBIOL., vol. 54, 2008, pages 1-17, XP002620175,

## Description

La présente invention concerne un complexe macromoléculaire d'origine bactérienne ainsi que l'utilisation dudit complexe macromoléculaire pour la prophylaxie et le traitement des rhumatismes inflammatoires.

De nombreuses études scientifiques ont mis en évidence le rôle que joue la flore intestinale dans la pathogénèse des maladies inflammatoires rhumatismales chroniques telles que la polyarthrite rhumatoïde, la spondylarthrite ankylosante ou les rhumatismes post-infectieux.

Par exemple, l'absence de signes d'arthrite est observée chez les animaux sans germes (rats ou souris transgéniques) alors que leurs confrères hébergeant une flore intestinale développent des signes d'arthrite (Rath HC,et al ; J.Clin.Invest ;98(4) ;945-953 ;1996 et Abdollahi-Roodsaz S., et al ; J.Clin.Invest ;118 ;205-216 ;2008**).**

Par ailleurs, chez l'être humain, des études ont montré que les patients dont la polyarthrite rhumatoïde était de diagnostic récent, hébergeaient peu de bifidobactéries, par rapport à des sujets témoins (Vaahtovuo J,et al ; J.Rheumatol ;35 ;690-693 ;2008) et lorsque l'équilibre de la flore intestinale était partiellement restauré par l'introduction d'une alimentation végétarienne, l'état du patient se trouvait être amélioré (Peltonen R, et al ; J.Rheumatol ;36 ;64-68 ;1997**).** Enfin, il est bien connu que l'utilisation de certains antibiotiques qui modifient la composition de la flore intestinale améliore les signes de la polyarthrite rhumatoïde (Stone M. et al. ; J.Rheumatol ;30 ;2112-2122 ;2003).

Un des mécanismes expliquant l'implication de la flore intestinale dans la pathogénèse des maladies rhumatismales chroniques réside dans sa capacité à réguler la translocation bactérienne. Le mécanisme de translocation bactérienne est défini par le passage de la barrière intestinale de bactéries intestinales. Ces bactéries intestinales sont captées puis transportées par des cellules du système immunitaire intestinal telles que des cellules dendritiques ou les macrophages vers le site synovial provoquant un foyer inflammatoire douloureux de type rhumatismal au niveau des articulations.

La composition de la flore intestinale influence ce processus. Ainsi, lorsque les bifidobactéries colonisent largement la partie basse de l'intestin, ces dernières montrent une capacité à réduire la translocation bactérienne (Romond MB, et al. ; Anaerobe ; 14 ; 43-48 ; 2008**).**

Par ailleurs, la composition de la flore intestinale influence également le niveau d'expression de gènes impliqués dans la réponse inflammatoire, tels que les galectines (Romond MB, et al ; Fems Immunol Med Microbiol ; 55 ; 85-92 ; 2009).

Il existe à ce jour de nombreux produits capables de modifier la flore intestinale tels que les prébiotiques ou les probiotiques. En revanche, peu d'entre eux ont un impact positif sur la translocation bactérienne. Parmi les produits ayant une action bénéfique vis-à-vis de la translocation bactérienne, on trouve la macromolécule isolée de culture de *Bifidobacterium breve.* En effet, il a été démontré dans les documents WO 2004/093898 et WO 2006/040485 que l'administration par voie orale de cette macromolécule entrainait une diminution de la translocation et de la dissémination bactérienne et que cette dernière présentait une activité préventive de l'arthrite induite par collagène chez la souris. Cependant, une activité pro-inflammatoire résiduelle est toujours observée avec l'utilisation de la macromolécule isolée de culture de *Bifidobacterium breve,* ce qui limite son utilisation dans le domaine des maladies inflammatoires.

Il serait donc avantageux de disposer d'un produit permettant une diminution de la translocation bactérienne et qui ne présenterait pas d'activité pro-inflammatoire résiduelle.

Or, la demanderesse a découvert que l'utilisation d'un complexe macromoléculaire isolé de culture de *Bifidobacterium longum* permettait de répondre à ces exigences.

Le complexe macromoléculaire de la présente invention est produit par la souche *Bifidobacterium longum* CBi0703, déposée selon le traité de Budapest sous le numéro CNCM I-3994 au nom de BIFINOVE, le 23 Mai 2008 auprès de la Collection Nationale de Cultures de Microorganismes (CNCM) tenue par l'Institut Pasteur, 25 rue du docteur Leroux 75015 Paris.

Ledit complexe macromoléculaire est constitué de chaînes associant une lipoprotéine et un oligosaccharide au sein desquelles :
- la lipoprotéine présente une masse moléculaire de 30kDa à 60kDa ;
- l'oligosaccharide présente une masse moléculaire inférieure à 15kDa, et de manière préférée inférieure à 10kDa ;
- la partie lipoprotéique qui est constituée de l'ensemble des lipoprotéines de chacune des chaînes représente 75 à 99%, préférentiellement de 80 à 98%, plus préférentiellement de 85 à 95% en poids de la masse totale du complexe et la partie oligosaccharidique qui est constituée par l'ensemble des oligosaccharides liée à chacune des chaînes représente 1 à 25%, préférentiellement de 2 à 20%, et plus préférentiellement de 5 à 15% de la masse totale du complexe.

Le complexe macromoléculaire comporte plusieurs chaînes associant une lipoprotéine et un oligosaccharide, lesdites chaînes étant de natures identiques ou différentes. Il est nécessaire selon l'invention, que le complexe macromoléculaire comportant plusieurs chaînes se présente sous forme agglomérée afin qu'un effet anti-arthritique soit observé. En effet, l'administration de structures monomériques de faibles masses moléculaires (typiquement de l'ordre de 15 à 50kDa) chez des souris a entraîné une élévation significative du score arthritique.

Le complexe macromoléculaire selon l'invention doit donc nécessairement présenter une masse moléculaire supérieure à 150kDa, de préférence supérieure à 400kDa et de manière particulièrement préférée supérieure ou égale à 600kDa.

Selon l'invention, la lipoprotéine comprend la séquence en acide aminés SEQ ID n°1:

La séquence en acides aminés de la lipoprotéine est SEQ ID n° 2 :

Les saccharides constituant la partie oligosaccharidique du complexe macromoléculaire de la présente invention peuvent être choisis parmi le galactose (Gal), la N-acétylgalactosamine (Gal Nac), le glucose (Glc), la N-acétylglucosamine (Glc Nac), le rhamnose (Rham), le mannose (Man) et leurs mélanges.

Selon l'invention, le ratio massique de sucre entrant dans la composition de la macromolécule est de 1 à 25%, de préférence de 2 à 20% et plus préférentiellement encore de 5 à 15%.

La composition massique moyenne de galactose est comprise entre 1 et 50µg/mg de complexe macromoléculaire, de préférence entre 5 et 20µg/mg de complexe macromoléculaire, de mannose est comprise entre 0,5 et 10µg/mg de complexe macromoléculaire, de préférence entre 1 et 10 µg/mg de complexe macromoléculaire ; celle de glucose est comprise entre 3 et 80µg/mg de complexe macromoléculaire, de préférence entre 5 et 50µg/mg de complexe macromoléculaire, et encore plus préférentiellement entre 10 et 50µg/mg de complexe macromoléculaire ; celle de N-acétylgalactosamine est comprise entre 2 et 30µg/mg de complexe macromoléculaire, de préférence entre 2 et 20µg/mg de complexe macromoléculaire, et encore plus préférentiellement entre 2 et 10µg/mg de complexe macromoléculaire ; celle de N-acétylglucosamine est comprise entre 1 à 10µg/mg de complexe macromoléculaire, de préférence entre 1 et 5µg/mg de complexe macromoléculaire ; celle de rhamnose est comprise entre 0,05 et 10µg/mg de complexe macromoléculaire, de préférence entre 0,05 et 5µg/mg de complexe macromoléculaire, et encore plus préférentiellement entre 1 et 5µg/mg de complexe macromoléculaire.

Les lipides constituant la partie lipidique du complexe macromoléculaire de la présente invention peuvent être choisis dans le groupe constitué des acides gras saturés longs en C14, C16, C18 et leurs mélanges.

Par ailleurs, il a été montré que cette structure macromoléculaire est reconnue par la galectine-1 et le TLR-6, ce qui indique d'une part que des unités de galactose sont en position externe accessible au récepteur galectine 1 (la galectine 1 reconnaissant le galactose présent dans le lactose, et surtout le galactose en position externe présent dans les glycoconjugués), et d'autre part que le complexe macromoléculaire conserve des unités de lipoprotéine reconnaissables par la sous-unité spécifique des lipoprotéines du complexe TLR2/6.

Les tests effectués par la Demanderesse ont montré que l'administration dudit complexe macromoléculaire induit une activité anti-arthritique chez la souris, dans un modèle d'arthrite induite au collagène.

Cette activité anti-arthritique a été mise en évidence à travers plusieurs observations :
- une amélioration de la réponse transcriptomique des cellules dendritiques à la translocation bactérienne chez la souris associée à un biotope de patient atteint d'arthrite ;
- un conditionnement de ces cellules dendritiques entraînant une co-évolution avec la population de lymphocytes T régulateur responsable de la production d'interleukine-10 (cytokine ayant un rôle important dans l'immunomodulation dans le système digestif et qui présente notamment des effets anti-inflammatoires) .

Le complexe macromoléculaire selon l'invention est obtenu par un procédé comportant les étapes suivantes :
(i) l'ensemencement et l'incubation pendant 16 à 60h, en conditions anaérobies et à une température comprise entre 30°C et 39°C environ, d'une souche de *Bifidobacterium longum* déposée selon le traité de Budapest sous le numéro CNCM I-3994 auprès de la Collection Nationale de Cultures de Microorganismes (CNCM) dans un milieu de culture et comprenant une fraction de protéines de lactosérum natives ou hydrolysées, du lactose et un agent anti-oxydant ;
(ii) la séparation desdites bactéries dudit milieu de culture;
(iii) l'ultrafiltration du surnageant sur des membranes de filtration ayant un seuil de coupure de 10 à 100 kDa, conduisant à l'obtention d'un rétentât concentré ;
(iv) l'enrichissement en complexe macromoléculaire par lavage avec un volume de 5 à 50 fois le volume du rétentât concentré ;
(v) la purification du complexe macromoléculaire par chromatographie de tamisage moléculaire en conditions stériles, par exemple sur gel Superdex 200 ;
(vi) la récupération de la fraction exclue qui comprend le complexe macromoléculaire bactérien.

Il est important de prévenir l'oxydation durant le procédé d'où la nécessité d'introduire un agent anti-oxydant tel que l'acide ascorbique, le chlorhydrate de cystéine ou le thioglycollate.

Selon un mode de réalisation, le milieu de culture contient en outre d'autres composés tels que le dihydrogénophosphate de potassium intervenant dans la stabilisation du pH.

Selon un mode de réalisation, l'ensemencement des bifidobactéries dans ledit milieu de culture peut se faire à partir d'un concentré congelé ou d'une pré-culture de 16-24h, qui permet la prolifération des bactéries.

Selon un autre mode de réalisation pouvant être combiné avec le précédent, les bactéries sont ensemencées dans ledit milieu de culture à raison de 10⁵-10¹⁰ unités formant colonies par mL de milieu.

Selon un mode de réalisation préféré de l'invention, le milieu de culture comporte 1 à 20g/L de milieu de protéines de lactosérum natives ou hydrolysées, 30 à 80g/L de milieu de lactose et 0,1 à 0,5g/L de milieu d'acide ascorbique. Selon un autre mode de réalisation préféré de l'invention, le milieu de culture comporte 1 à 20g/L de milieu de protéines de lactosérum natives ou hydrolysées, 30 à 80g/L de milieu de lactose et 0,1 à 0,5g/L de milieu d'acide ascorbique et 0,5 et 3g/L de milieu de dihydrogénophosphate de potassium.

Selon un mode de réalisation pouvant être combiné avec les précédents, le pH dudit milieu de culture n'est pas régulé pendant l'incubation.

Enfin, selon un autre mode de réalisation, le pH dudit milieu de culture est maintenu entre 4 et 7 pendant l'incubation.

L'invention porte sur l'utilisation du complexe macromoléculaire d'origine bactérienne selon l'invention pour prévenir et traiter les troubles articulaires, pour la régulation de la flore intestinale et de la translocation bactérienne.

Selon un autre aspect, la présente invention a pour objet l'utilisation dudit complexe macromoléculaire d'origine bactérienne obtenu selon le procédé décrit précédemment dans des produits destinés à l'industrie pharmaceutique, agroalimentaire et/ou nutraceutique.

L'invention a notamment pour objet une composition pharmaceutique comprenant au moins ledit complexe macromoléculaire, à titre de principe actif, et au moins un support pharmaceutiquement acceptable.

La concentration massique dudit complexe macromoléculaire représente de 0,1µg/g à 50µg/g de la composition pharmaceutique.

Par pharmaceutiquement acceptable, on entend tout support qui permet non seulement de conserver les propriétés immunomodulatrices du complexe macromoléculaire obtenu selon le procédé décrit précédemment, mais aussi de véhiculer ledit complexe macromoléculaire.

L'utilisation de la composition pharmaceutique permet de réguler la flore intestinale et la translocation bactérienne. Elle est par conséquent destinée à la prophylaxie et au traitement des rhumatismes inflammatoires tels que la polyarthrite rhumatoïde et la spondylarthrite ankylosante, de l'arthrose.

L'invention porte donc sur l'utilisation de la composition pharmaceutique pour l'obtention d'un médicament destiné à réguler la flore intestinale et la translocation bactérienne.

Ainsi, l'invention concerne une composition pharmaceutique pour une utilisation dans la régulation de la flore intestinale et la translocation bactérienne.

L'invention porte aussi sur l'utilisation de la composition pharmaceutique pour l'obtention d'un médicament destiné à traiter ou à prévenir les rhumatismes inflammatoires, l'arthrose.

Ainsi, l'invention concerne une composition pharmaceutique pour une utilisation dans le traitement et la prévention des rhumatismes inflammatoires, de l'arthrose

L'invention porte également sur l'utilisation de la composition pharmaceutique pour l'obtention d'un médicament destiné à traiter la polyarthrite rhumatoïde et la spondylarthrite ankylosante, l'arthrose et la fibromyalgie.

Ainsi, l'invention concerne une composition pharmaceutique pour une utilisation dans le traitement de la polyarthrite rhumatoïde et de la spondylarthrite ankylosante, de l'arthrose. La composition pharmaceutique de la présente invention peut se présenter sous toute forme galénique souhaitée pour une administration soit par voie orale à l'homme ou à l'animal comme par exemple sous forme liquide (sirop, solution, spray) ou sous forme solide (poudre, comprimé, gélule, capsule, spray poudre , gomme, pâte, granulés, dans leurs formes diverses, à libération immédiate ou programmée), ou par voie rectale, nasale, pulmonaire, parentale ou sous une forme adaptée à une administration par inhalation ou insufflation. Le mode d'administration préféré est généralement la voie orale.

La composition pharmaceutique comprenant ledit complexe macromoléculaire peut être conservée à une température de -70°C à +4°C pour les formes liquides et jusqu'à +40°C pour les formes solides pendant 3 ans.

Par ailleurs, le complexe macromoléculaire obtenu selon le procédé décrit précédemment peut également être incorporé, à titre d'ingrédient, dans des compositions alimentaires.

Par conséquent, l'invention a également pour objet une composition alimentaire comprenant au moins le complexe macromoléculaire et au moins un ingrédient alimentaire. L'ingrédient alimentaire peut être par exemple une préparation lactée, des céréales, etc.

Une telle composition alimentaire peut être destinée à l'homme ou à l'animal et peut notamment se trouver sous la forme de produits alimentaires diététiques ou non, à usage hospitalier ou non. En particulier, cette composition peut être un soluté entéral.

La concentration massique dudit complexe macromoléculaire représente de 10µg/g à 2µg/g, de préférence de 10µg/g à 1µg/g de la composition alimentaire.

Enfin, le complexe macromoléculaire obtenu selon le procédé décrit précédemment peut être incorporé à titre d'ingrédient alimentaire dans des compositions nutraceutiques.

L'invention a donc comme objet une composition nutraceutique comprenant au moins ledit complexe macromoléculaire, et au moins un support nutraceutiquement acceptable.

La concentration massique dudit complexe macromoléculaire représente de 10µg/g à 5µg/g de la composition nutraceutique.

Par composition nutraceutique, on entend une composition qui possède des effets physiologiques bénéfiques ou protecteurs supérieurs à ceux que pourrait apporter la nutrition classique.

Une telle composition nutraceutique peut se trouver sous la forme de compléments alimentaires. Ces compléments alimentaires peuvent être sous forme solide tels que des comprimés, poudres, gélules, capsules, ou sous forme liquide telles que des boissons ou des émulsions.

La composition nutraceutique selon l'invention peut donc être utilisée afin de prévenir les rhumatismes inflammatoires, l'arthrose.

L'invention porte donc sur l'utilisation de ladite composition nutraceutique pour l'obtention d'un complément alimentaire destiné à prévenir les rhumatismes inflammatoires, l'arthrose.

Ainsi, l'invention porte sur une composition nutraceutique pour une utilisation dans la prévention des rhumatismes inflammatoires, de l'arthrose.
La figure 1 représente la reconnaissance in vitro par la galectine-1 du complexe macromoléculaire obtenu soit avec une fermentation à pH régulé ou à pH non régulé selon l'invention.

La figure 2 représente la reconnaissance in vitro par le TLR-6 du complexe macromoléculaire obtenu soit avec une fermentation à pH régulé ou à pH non régulé selon l'invention.

La figure 3 représente le score d'arthrite chez des souris DBA1 après deux injections de collagène avec ou sans traitement par le complexe macromoléculaire préparé selon l'exemple 2 (0,2mg/L, soit 30-40 µg/kg). La figure 4 représente l'évolution de l'arthrite induite par un cocktail d'anticorps et du LPS chez des souris DBA1.

La figure 5 représente l'évolution du score d'arthrite chez les souris DBA1 traitées par le complexe macromoléculaire hydrolysé (0,2mg de complexe macromoléculaire hydrolysé/L).
La figure 6a représente des articulations de membres postérieurs gauches (n'ayant pas subi d'injection de iodoacétate de sodium : MIA) de rats appartenant au groupe témoin non traité par le complexe macromoléculaire de l'invention.
La figure 6b représente des articulations de membres postérieurs gauches (n'ayant pas subi d'injection de MIA) de rats appartenant au groupe traité par le complexe macromoléculaire de l'invention.
Les figures 7a et 7b représentent des articulations de membres postérieurs droits (ayant subi une injection de MIA) de rats appartenant au groupe témoin non traité par le complexe macromoléculaire de l'invention.
Les figures 7c et 7d représentent des articulations de membres postérieurs droits (ayant subi une injection de MIA) de rats appartenant au groupe traité par le complexe macromoléculaire de l'invention.

La présente invention sera illustrée par les exemples suivants.

### EXEMPLES

### Exemple 1 Préparation et isolement du complexe macromoléculaire selon l'invention (pH régulé pendant la fermentation)

Un milieu de culture contenant les ingrédients suivants est préparé :
- 1 à 20g/L d'une base protéique composée de perméat de protéines de lait hydrolysé ou sous forme native
- 30 à 80g/L de lactose
- 0,1 à 0,5g/L d'acide ascorbique
- 0,5 à 3g/L de dihydrogénophosphate de potassium

Une première solution est reconstituée avec le seul lactose et stérilisée à 108°C pendant 130 minutes. Une deuxième solution est reconstituée avec le reste des ingrédients et stérilisée à 121°C pendant 20 minutes.

La fermentation est de type discontinu à pH régulé pendant 24 heures.

### 1- Fermentation de type discontinu à pH régulé

Le pH est ajusté à une valeur de 6,5 une fois les deux solutions versées dans le fermenteur. Le milieu de culture est ensemencé avec 6 à 10% (v/v) d'un inoculum de 24h contenant entre 1x10⁶ à 2x10⁸ unités formant colonies (UFC) de bifidobactéries issues de la souche *Bifidobacterium longum* déposée sous le numéro CNCM I-3994 auprès de la Collection Nationale de Cultures de Microorganismes (CNCM)par mL de milieu de culture. Les bactéries sont cultivées sous agitation, sans aération du milieu et à une température de 37°C. Le pH est maintenu à 6,5 par ajout de soude (1N à 3 N) lors de la fermentation.

La fermentation dure 24 heures et la population de *Bifidobacterium longum* en fin de culture est comprise entre 2x10⁸ à 1x10¹⁰ UFC par mL de milieu de culture.

### 2- Isolement du complexe macromoléculaire

En fin de culture, les bactéries sont éliminées par centrifugation à 13000g pendant 30 minutes à une température de 4°C ou par microfiltration sur cassettes MILLISTACK (Millipore) de surface appropriée au volume de fermentation.

Le surnageant est ultrafiltré à température ambiante et en condition stérile sur un appareil type AMICON Proflux M12 (Millipore). Une première ultrafiltration est effectuée sur une cartouche spiralée type HELICON (Millipore) d'un seuil de coupure de 10Da.

Le surnageant est concentré au maximum 15 fois, puis lavé en continu avec de l'eau osmosée stérile, 30 fois le volume concentré. Le rétentat de 10 kDa récupéré est conservé à 4°C ou congelé pour la seconde ultrafiltration.

La seconde ultrafiltration est réalisée également à température ambiante et en conditions stériles sur le même appareil que précédemment mais équipé d'un support pour cassettes type PELLICON (Millipore). La cassette utilisée est de type BIOMAX de 0,5 m² de surface et d'un seuil de coupure de 100 kDa. Le rétentat de 10 kDa est concentré au maximum 2 fois puis lavé en continu avec 30 volumes d'eau osmosée.

Le rétentat >100 kDa est conservé sous forme congelée ou lyophile jusqu'à purification.

Le complexe macromoléculaire contenu dans le rétentat >100 kDa, est ensuite obtenu en conditions stériles par chromatographie d'exclusion sur un gel type Superdex 200 (GE Healthcare). Le complexe macromoléculaire est ainsi séparé puis élué dans la fraction exclue (> 600 kDa) avec un tampon Tris/HCl 50 mM - 150 mM NaCl , pH 8.0.

Cette fraction exclue est ensuite dessalée par filtration, puis diafiltrée avec 5 à 7 volumes d'eau osmosée stérile. Le complexe macromoléculaire est ainsi récupéré et conservé sous une forme lyophile.

### Exemple 2 : Préparation et isolement du complexe macromoléculaire selon l'invention (pH non régulé pendant la fermentation)

La composition du milieu de culture et les étapes sont les mêmes que dans l'exemple 1 à l'exception que dans cet exemple le pH n'est pas régulé pendant la fermentation, l'étape 1 est ainsi remplacée par l'étape décrite ci-dessous.

### 1-Fermentation de type discontinu à pH non régulé lors de la fermentation :

Le pH est ajusté à une valeur de 6,5 une fois les deux solutions versées dans le fermenteur. Le milieu de culture est ensemencé avec 6 à 10 % (v/v) d'un inoculum de 24h, contenant entre 1x10⁶ à 2x10⁸ unités formant colonies (UFC) de bifidobactéries issues de la souche *Bifidobacterium longum* déposée sous le numéro CNCM I-3994 auprès de la Collection Nationale de Cultures de Microorganismes (CNCM) par mL de milieu de culture. Les bactéries sont cultivées sous agitation, sans aération du milieu et à une température de 37°C. Le pH n'est pas ajusté pendant la fermentation. La fermentation dure 24 heures et la population de *Bifidobacterium longum* en fin de culture est comprise entre 1x10⁷ à 1x10⁹ UFC par mL de milieu de culture.

L'isolement du complexe macromoléculaire peut être ensuite réalisé selon l'étape 2 de l'exemple 1.

Dans la suite de ce texte, C1 fait référence au complexe macromoléculaire préparé selon l'exemple 2 de l'invention.

### Exemple 3 Caractérisation du complexe macromoléculaire

Les parties saccharidiques, protéiques et lipidiques dont est constitué le complexe macromoléculaire ont été caractérisées qualitativement et quantitativement à l'aide de diverses méthodes analytiques.

La concentration protéique a été déterminée par la méthode de Lowry. Elle est de 80 à 400 µ/mg de poudre (8 à 40 % du complexe macromoléculaire).

La concentration en saccharides a été déterminée par Chromatographie en Phase Gazeuse après méthanolyse (MeOH/HCl à 0,5 N), puis dérivation par anhydride heptafluorobutyrique (20 µL dans 100 µL d'acétonitrile anhydre). Elle est de 20 à 150 µg/ mg de poudre (2 à 15 % du complexe macromoléculaire).
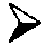 lipoprotéine : la séquence SEQ ID n° 2 est la suivante :
   De plus, la composition en lipide établie par chromatographie en phase gazeuse couplée à la spectrométrie de masse montre la présence de pics majeurs d'acides gras de longueur C14:0, C16:0 et C18:0.
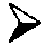 sucre: la partie saccharidique de la macromolécule contient les saccharides cités dans le tableau 1 selon les ratios molaires suivants:

**Tableau 1: Répartition massique de sucres entrant dans la composition du complexe macromoléculaire**

| | ***Gal*** | ***Man*** | ***Glc*** | ***Gal NAc*** | ***Glc NAc*** | ***Rham*** |
|---|---|---|---|---|---|---|
| *Composition massique (µg*/*mg de complexe macromoléculaire)* | 15,35 | 4,95 | 35, 10 | 6, 75 | 1, 5 | 1,45 |

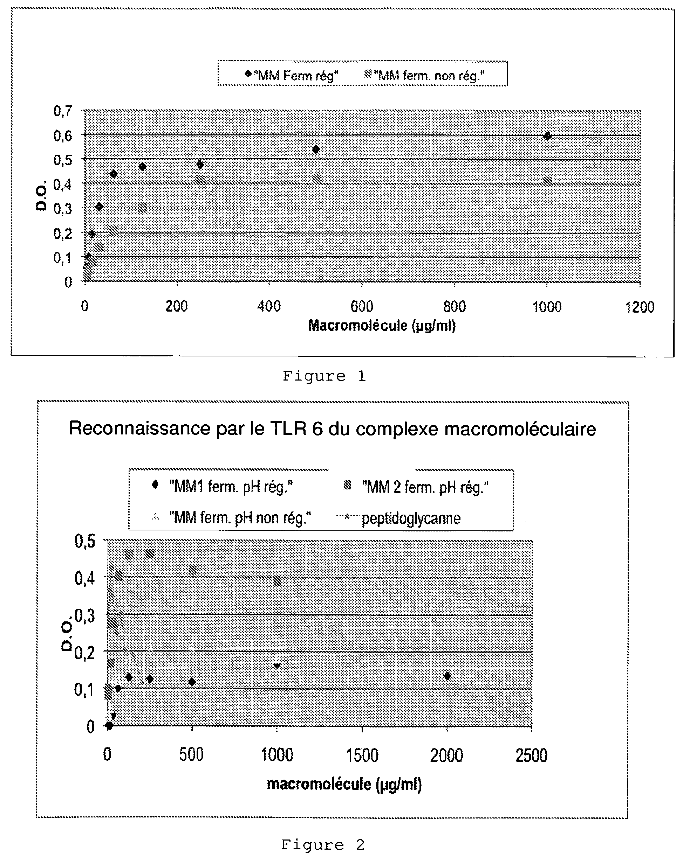

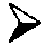 Le complexe macromoléculaire est reconnu in vitro par la galectine 1 (figure 1).
   Dans le présent exemple, le complexe macromoléculaire de la présente invention et issu de la fermentation à pH régulé ou non régulé est reconnu, après adsorption dans des cupules de microplaques par la galectine 1 (révélation par système biotine-streptavidine). Selon la figure 1, la saturation est observée pour des concentrations de l'ordre de 10 à 20 µg de complexe macromoléculaire pour 10 ng de galectine-1.
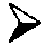 Le complexe macromoléculaire est reconnu in vitro par le TLR-6 (figure 2)
   Dans le présent exemple, le complexe macromoléculaire de la présente invention issu de fermentation à pH régulé ou non régulé est reconnu, après adsorption dans des cupules de microplaques, par le TLR6 (révélation par système biotine-streptavidine). Selon la figure 2, la saturation est observée pour des concentrations de l'ordre de 10 à 20 µg de macromolécule pour 10 ng de TLR6. L'expression par unité de peptidoglycane est environ de 0,2 à 0,4 µg de peptidoglycane pour 1 µg de macromolécule.

### Exemple 4 : Effet du complexe macromoléculaire C1 chez la souris à flore de patient arthritique

Pour cette étude, les souris utilisées ont été divisées en deux groupes :
- un groupe essai dans lequel des souris axéniques ont été associées à la flore de patient atteint de polyarthrite évolutive (FA) ;
- un groupe témoin dans lequel des souris axéniques ont été ensemencées avec la flore d'un volontaire sain (FN).

Les descendants de la première à la quatrième génération ont été utilisés pour l'étude.

Le complexe macromoléculaire C1 [pH non régulé] a été administré par voie orale pendant 15 jours à la dose de 0,2mg/L (la dose moyenne prise par souris est comprise entre 25-50µg/kg).

Les souris, à jeun depuis la veille, ont été euthanasiées. La rate a été prélevée aseptiquement et traitée par collagénase pour libérer les splénocytes. Les cellules dendritiques CD11c, les lymphocytes Th CD4(+) et Trég. ont été isolés à l'aide du système MACs. La flore intestinale a été analysée dans l'iléon, le caecum et le côlon. La translocation bactérienne a été évaluée par analyse bactériologique des plaques de Peyer, du foie, du poumon, des reins et du sang.

Les résultats sont regroupés dans le tableau 2 et mettent clairement en évidence une augmentation du taux de bifidobactéries le long de l'intestin des souris traitées avec C1 et montre ainsi l'effet bénéfique de l'administration de ce dernier sur la flore intestinale.

**Tableau 2 : Effet de l'administration du complexe macromoléculaire C1 sur la flore intestinale.**

| | témoin | essai | p |
|---|---|---|---|
| Souris (n) | 6 | 5 | |
| *iléon* | | | |
| E.coli | 5,38 ± 0,6 (5) | 4,89 ± 1,3 (5) | NS |
| Bifidobactéries | 2,68 ± 0,7 (2) | **3,49 ± 1,7 (5)** | 0,0398 |
| | | | |
| *caecum* | | | |
| E.coli | 7,89 ± 0,3 (6) | 7,52 ± 0,4 (5) | NS |
| Bifidobactéries | 3,06 ± 0,4 (3) | **6,43 ± 1,5 (5)** | 0,0061 |
| | | | |
| *côlon* | | | |
| E.coli | 7,61 ± 1,4 (6) | 8,11 ± 0,4 (5) | NS |
| Bifidobactéries | 3,0 ± 0,8 (6) | **6,56 ± 1,5 (5)** | 0,0057 |

### Exemple 5 : Implication des bactéries intestinales dans l'arthrite chez les souris DBA1 et effet du complexe macromoléculaire C1 de l'invention

Pour cette étude, deux modèles ont été établis selon le type d'injection choisie :
- Modèle 1 : deux doses de collagène à trois semaines d'intervalle sont injectées chez des souris DBA1 ;
- Modèle 2 : un cocktail d'anticorps anti-collagène (MD Biosciences) suivi d'une injection trois jours plus tard de LPS (lipopolysaccharide) sont injectés chez des souris DBA1.

Quelque soit le modèle, ces injections induisent un tableau clinique évocateur de la PAR (polyarthrite rhumatismale). Le score d'arthrite est établi en estimant la rougeur et le gonflement des pattes.

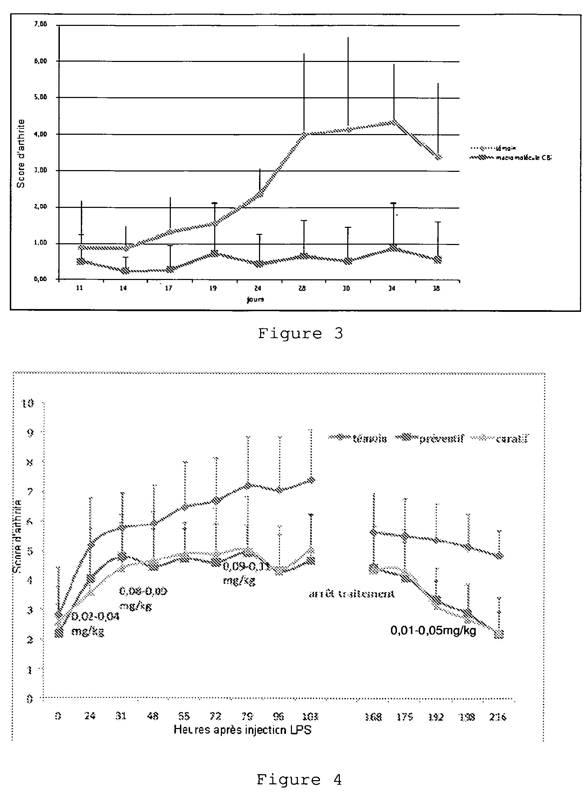

Le traitement par le complexe macromoléculaire C1 est initié soit 15 jours avant la première injection de l'inducteur (modèle 1-figure 3), soit à l'apparition des symptômes (modèle 2-figure 4). Les concentrations du complexe macromoléculaire C1 s'échelonnent entre 0,1mg/L et 0,5 mg/L, soit des doses journalières estimées entre 10 et 100µg/kg de poids corporel.

La dose efficace est située dans une fourchette de 30 à 90 µg/kg selon le modèle d'induction de l'arthrite (figures 3 et 4).

Le score augmente environ dix jours après la deuxième injection de collagène dans le premier modèle et 3-4 jours après l'injection de LPS dans le deuxième cas.

Après trois jours d'arrêt (modèle 2), le traitement est à nouveau administré (à raison de 0,2 mg/L, soit 0,01 à 0,05 mg du complexe macromoléculaire C1 /kg de poids corporel).

Le tableau 3 représente l'analyse concomitante de la flore intestinale et montre que l'intensité de l'inflammation articulaire est plus intense lorsque le nombre de cellules dendritiques spléniques est faible sous traitement par le complexe macromoléculaire C1 de l'invention, alors qu'il n'y a pas de dépendance entre les deux facteurs en absence de traitement.

Or, on observe chez les souris traitées que le nombre de cellules dendritiques spléniques est lié non seulement à une population de lactobacilles (Lb09) mais aussi à la population de *Bacteroides* caecaux. En absence de traitement, le nombre de cellules dendritiques est affecté exclusivement par des populations de lactobacilles. Dans les deux groupes de souris, plus le nombre de *Lactobacillus* 09 est élevé dans le côlon, moins la rate héberge des cellules dendritiques. Mais la corrélation entre *Lactobacillus* 09 et intensité de l'inflammation articulaire ne devient significative que lorsque le traitement annule l'effet d'une deuxième population de lactobacilles *(Lactobacillus* 081) sur les cellules dendritiques (tableau 3).

On remarque donc une corrélation entre cette population de lactobacilles (Lb 081) localisée dans le caecum et les plaques de Peyer et l'intensité de l'inflammation en absence de traitement alors qu'aucune dépendance n'est retrouvée sous traitement bien que la population de *Lactobacillus* 081 ne diminue pas (tableau 3).

En conclusion, le traitement n'a donc pas d'effet antibactérien vis-à-vis de la population lactobacillaire, mais influence le bilan métabolique des lactobacilles Lb081, dont un ou plusieurs des produits finaux pourraient être impliqués dans l'inflammation articulaire.

**Tableau 3 : Corrélation entre populations bactériennes intestinales, score d'arthrite et cellules dendritiques selon le traitement**

| souris témoin | | rs* | p |
|---|---|---|---|
| localisation | Bactéries | vs score d'arthrite | |
| Intestin grêle | *Lactobacillus reuteri* | -0,736 | <0,04 |
| | *Streptococcus sp.* | -0,812 | <0,02 |
| Caecum | *Lactobacillus sp. 081* | 0,88 | <0,01 |
| | *Bacteroides fragilis* | -0,862 | <0,006 |
| Côlon | *Streptococcus sp.* | -0,986 | <0,0004 |
| | | | |
| Plaques de Peyer | *Lactobacillus sp. 081* | 0,88 | <0,01 |
| | *Streptococcus sp.* | -0,736 | <0,05 |
| | | vs CD spléniques | |
| Intestin grêle | *Lactobacillus sp. 081* | 0,771 | <0,05 |
| Côlon | *Lactobacillus sp. 09* | -0,829 | <0,01 |
| | | | |

| Souris traitée par le complexe macromoléculaire | | rs* | |
|---|---|---|---|
| localisation | | vs score d'arthrite | |
| | | | |
| Caecum | *Bacteroides sp.* | -0.791 | <0,05 |
| Côlon | *Lactobacillus sp. 09* | 0,805 | <0,02 |
| Plaques de Peyer | *Lactobacillus sp.* 082 | -0,77 | <0,02 |
| | | vs CD spléniques | |
| Caecum | *Bacteroides fragilis* | 0,829 | <0,05 |
| Côlon | *Lactobacillus sp. 09* | -0,829** | <0,05 |

| | | | |
|---|---|---|---|
| *rs=coefficient de Spearman **corrélation score d'arthrite en fonction du nombre de cellules dendritiques rs=-0,812 p<0,05 | | | |

### Exemple 6 : Importance de l'agrégation des chaînes constituant le complexe macromoléculaire C1 de l'invention

Cet exemple met en évidence l'effet de l'hydrolyse acide sur l'efficacité anti-arthritique.

28 mg du complexe macromoléculaire préparé selon l'exemple 2 sont dilués dans 100 mL d'une solution d'acide acétique à 70 %. La solution est portée à 75°C pendant 100 min. La dégradation en molécules de faible masse moléculaire est vérifiée par chromatographie sur Superdex 200 (la concentration de macromolécule résiduelle est de l'ordre de 2,5%). Après lyophilisation du produit dégradé qui permet l'élimination de l'acide acétique, la poudre est reprise dans un volume d'eau stérile équivalent pour administration à des souris DBA1 (modèle 1).

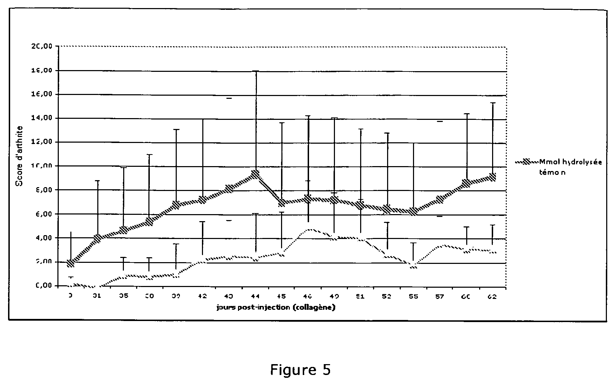

Selon la figure 5, on observe un effet pro-arthritique, les scores d'arthrite étant plus élevés au cours du temps dans le groupe traité par la macromolécule hydrolysée (ANOVA deux facteurs mesures répétées, p=5,43852E-08). En conclusion, il est nécessaire que les unités de lipoprotéine bifide associées aux oligosaccharides soient agrégées sous forme de macromolécules pour que l'activité anti-arthritique soit observée.

### Exemple 7 : Comparaison entre l'effet de l'administration du complexe macromoléculaire issu de la souche Bifidobacterium breve et celui issu de la souche Bifidobacterium longum

Le tableau 4 met en évidence l'effet du traitement par le complexe macromoléculaire issu de la souche *Bifidobacterium longum (C1)* d'une part et compare ce dernier à celui produit par l'administration du complexe macromoléculaire issu de la *Bifidobacterium breve* (tel qu'il est décrit dans WO 2006/040485).

Dans la suite de ce texte, C2 fait référence au complexe macromoléculaire préparé selon l'exemple 1 de WO 2006/040485.

Le protocole expérimental est identique à celui décrit dans l'exemple 4.

Les résultats regroupés dans le tableau 4 montrent que :
- le nombre de lymphocytes Th CD4(+) spléniques est plus faible chez les souris traitées par C2 (complexe macromoléculaire issu de la souche Bifidobacterium breve) par rapport aux souris témoins ou à celles traitées avec C1 (complexe macromoléculaire issu de la souche Bifidobacterium longum)
- le nombre de lymphocytes Treg (cellules régulatrices) est plus faible chez les souris traitées par C2 par rapport aux souris témoins ou à celles traitées avec C1.

Ces résultats montrent ainsi que le traitement est moins efficace dans le cas de l'administration du complexe macromoléculaire issu de la souche *Bifidobacterium breve* qu'avec le complexe macromoléculaire issu de la souche *Bifidobacterium longum* déposée sous le numéro CNCM I-3994. En effet, l'administration du complexe selon l'invention maintient la production de cellules T régulatrices, cellules jouant un rôle prépondérant dans la réponse anti-inflammatoire.

**Tableau 4 : Population cellulaire en fonction du traitement administré**

| FA | Cellules dendritiques | Lymphocytes | T |
|---|---|---|---|
| | | Th | Trég |
| souris (n)* | CD11c | CD4+ | CD4+, CD25+ |
| traitement témoin (4) | 3,6 ± 0,9** | 5,30 ± 0,6 | 0,32 ± 0,04 |
| C1 (4) | 3,04 ± 0,3 | 5,2 ± 0,3 | 0,30 ± 0,07 |
| C2 (4) | 3,28 ± 0,6 | 4,07 ± 1,0 | 0,22 ± 0,03 |

| | | | |
|---|---|---|---|
| * les souris sont d'âge comparable (environ 7 mois) a: expression en % de splénocytes **souris associées à flore de volontaire sain CD11c= 4,76 ± 1,6 | | | |

Par ailleurs, le tableau 5 représente la corrélation de Spearman entre les populations cellulaires.

Il est mis en évidence dans ce tableau que les taux de cellules dendritiques et de lymphocytes T spléniques sont corrélés chez les souris traitées avec le complexe macromoléculaire issu de la souche *Bifidobacterium longum* **C1** (corrélation de Spearman proche de 1) alors que les populations cellulaires sont numériquement indépendante chez les souris traitées avec le complexe macromoléculaire issu de la souche *Bifidobacterium breve* **C2** (corrélation de Spearman proche de 0).

Plus spécifiquement le taux de cellules dendritiques chez les souris traitées avec le complexe macromoléculaire issu de la souche *Bifidobacterium longum* **C1** est corrélé à la sous-population de lymphocytes T, les lymphocytes Treg CD4, CD25 producteur de l'interleukine 10 anti-inflammatoire. Les deux populations cellulaires évoluent donc de façon dépendante sous traitement avec le complexe macromoléculaire issu de la souche *Bifidobacterium longum* **C1.** Le complexe macromoléculaire issu de la souche *Bifidobacterium longum* **C1** conditionne donc les cellules dendritiques ce qui facilite le recrutement par ces cellules des lymphocytes producteurs d'interleukine 10.

**Tableau 5 : corrélation de Spearman entre les populations cellulaires selon le traitement**

| rs* | CD11c vs Th CD4+ | p | CD11c vs Th CD4+, CD25+ | p |
|---|---|---|---|---|
| Traitement témoin (9) | 0,45 | NS | 0, 45 | NS |
| C1(7) | **0,81** | <0,05 | 0, 79 | < 0.05 |
| C2 (7) | **0,16** | NS | 0,07 | NS |

### Exemple 8 : Modification de l'expression des gènes

Les tableaux 6 et 7 mettent en évidence l'effet du traitement lié à l'administration du complexe macromoléculaire C1 vis-à-vis de l'expression des gènes en comparant les expressions des gènes des cellules dendritiques de souris associées à une flore de patient arthritique par rapport à des souris associées à la flore de volontaire sain **(FA vs FN** dans le tableau 6) avec ceux des cellules dendritiques de rate de souris associée à une flore de patient arthritique traitées pendant 15 jours par 0,2mg/L du complexe macromoléculaire C1 par rapport à des souris non traitées **(FAt vs FA** dans tableau 6). Ces résultats montrent que l'hyperstimulation de la flore sur le système immunitaire est remise à la normale avec le traitement.

En effet, dans le cas de souris hébergeant une flore d'arthrite, un certain nombre de gènes sont surexprimés tels que des gènes de protéolyse ou d'hydrolyse des sucres intervenant dans la digestion des antigènes captés par les cellules dendritiques ainsi que des gènes impliqués dans l'inflammation (élastase). Après traitement (FAt vs FA), on retrouve une expression similaire à celle de souris hébergeant une flore de sujet sain: à titre d'exemple pour l'élastase, le rapport FC de son expression chez des souris hébergeant la flore d'arthrite **(FA)** vs des souris hébergeant une flore de sujet sain **(FN) (FA vs FN avant traitement)** est de + 24.725. Après traitement des souris hébergeant la flore d'arthrite par le complexe macromoléculaire C1, le rapport FC comparant son expression à celle des souris hébergeant la flore de patient atteint d'arthrite non traitées **(FAt vs FA) (après traitement)** est de -23.92, soit un retour à son expression de base chez une souris hébergeant une flore de sujet sain.

Par ailleurs, le tableau 7 met en évidence les gènes qui n'ont pas été suractivés par la flore d'arthrite par rapport à la flore de sujet sain mais dont l'expression est tout de même modifiée par l'administration du traitement avec la C1. Par exemple, le gène de la prostaglandine 2 reductase est sous-exprimé ce qui suggère une atténuation de l'inflammation. De même, le gène Ednrb impliqué dans les processus inflammatoires est sous-exprimé suite au traitement.

### Exemple 9 : Prévention de l'arthrose chimio-induite chez le rat par administration du complexe macromoléculaire Mode opératoire :

L'arthrose est induite chez des rats de souche CD mâles de 125-150g (CHARLES RIVER) par une injection dans l'articulation tibio-fémorale droite de 3 mg (0.05 ml d'une solution à 60mg/ml) d'iodoacétate de sodium (MIA).

Les animaux sont ensuite observés (gradation de la boiterie) pendant une période post-opératoire d'environ 15 jours avant de recevoir en continu le complexe macromoléculaire issu de la souche Bifidobacterium longum **C1** (solution aqueuse de 0,3mg de complexe macromoléculaire issu de la souche Bifidobacterium longum **C1**/L) per os pendant 3 semaines (période d'adaptation).

Pendant les 5 semaines suivantes, la même solution de complexe macromoléculaire issu de la souche Bifidobacterium longum **C1** est administrée de façon discontinue (alternance entre eau pendant 3-4 jours et administration de la solution contenant le complexe macromoléculaire **C1** pendant 3-4 jours). Ainsi, les doses reçues par les rats pendant la période d'adaptation sont de 26,3 ± 7 µg de complexe macromoléculaire **C1**/Kg de poids corporel, puis pendant la période d'administration des 5 semaines suivantes, de 18,9 ± 6 µg de complexe macromoléculaire **C1**/Kg de poids corporel.

A l'issu de l'essai, les rats sont euthanasiés par injection intra-péritonéale de pentobarbital sodique (CEVA Santé animale) 2 à 4 heures après avoir enlevé la boisson et l'alimentation.

Par ailleurs, des rats n'ayant pas reçu l'administration du complexe macromoléculaire issu de la souche Bifidobacterium longum **C1** sont également euthanasiés (groupe témoin).

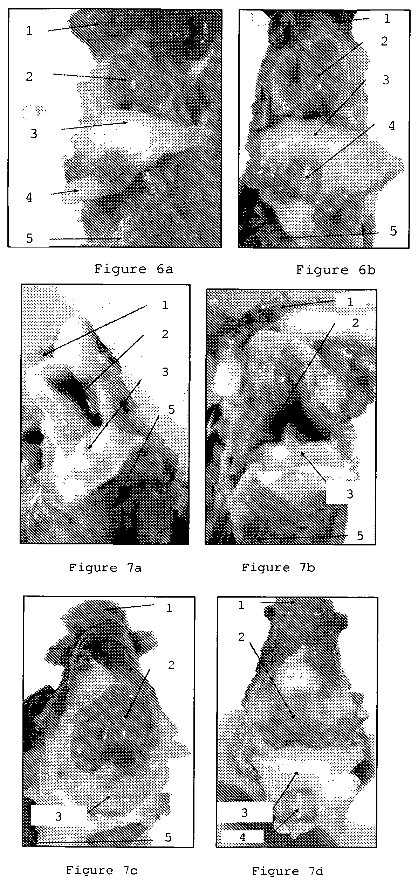

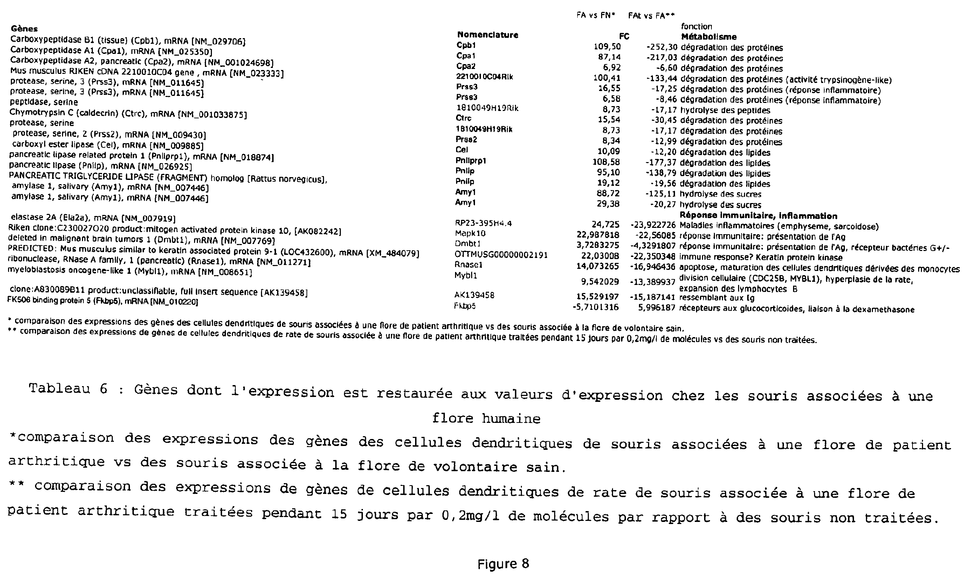

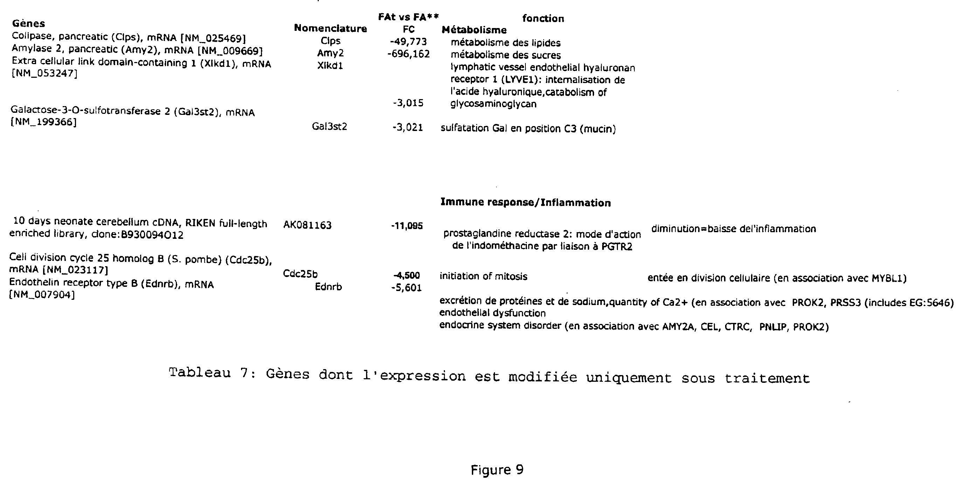

Les articulations des membres postérieurs des rats appartenant au groupe traité avec le complexe macromoléculaire issu de la souche Bifidobacterium longum **C1** et les rats du groupe témoin sont prélevées et la jonction articulaire tibio-fémorale est observée (Figures 6 et 7).

Sur les figures 6 et 7, 1 représente le fémur, 2 représente le cartilage, 3 représente la membrane synoviale, 4 représente la patella et 5 représente le tibia.

De plus, une analyse bactériologique est réalisée sur les organes suivants: sang, Poumon, Foie, rate, rein, Plaques de Peyer, iléon (trois fragments), caecum, côlon.

### Résultats :

Les figures 6a et 6b ne mettent en évidence aucune déformation des articulations des membres postérieurs. Les membres postérieurs droits (injection de MIA) des rats non traités par le complexe macromoléculaire C1 montrent une déformation évocatrice des lésions d'arthrose (Figures 7a et 7b). Au contraire, on ne note pas de déformation chez des rats ayant reçu le complexe macromoléculaire C1 (50% de rats répondeurs) (Figures 7c et 7d).

Par ailleurs, l'analyse bactériologique permet de formuler les observations suivantes. Chez les rats répondeurs, la flore intestinale est modifiée dans plusieurs sites :
- les staphylocoques sont indétectables dans l'iléon distal,
- les entérobactéries (autres que E.coli) sont indétectables dans le côlon,
- une diminution de clostridiies est observée dans le caecum (<2.7 vs 3.2 ± 0,5 logcfu/g chez les rats témoins),
- une diminution d'entérocoques est observée dans le côlon (<5 log cfu/g vs 6.2 ± 0.5 log cfu/g chez les rats témoins).

Par ailleurs on note une translocation de lactobacilles dans le poumon (4.2 ± 2.5 log cfu/g) alors que les poumons des rats non protégés sont stériles ou sont contaminés par des staphylocoques.

### Conclusions :

Lorsque l'administration du complexe macromoléculaire de l'invention modifie la balance de la flore intestinale dans le modèle d'arthrose et la translocation bactérienne, elle protège efficacement l'animal du processus dégénératif induit par l'injection de MIA.

## Revendications

1. Complexe macromoléculaire bactérien produit par des bactéries appartenant à la souche *Bifidobacterium longum,* déposée selon le traité de Budapest sous le numéro CNCM I-3994, le 23 Mai 2008 auprès de la Collection Nationale de Cultures de Microorganismes (CNCM), constitué de chaînes associant une lipoprotéine et un oligosaccharide **caractérisé par le fait que** :
- la lipoprotéine présente une masse moléculaire de 30kDa à 60kDa ;
- l'oligosaccharide présente une masse moléculaire inférieure à 15kDa;
- la partie lipoprotéique qui est constituée de l'ensemble des lipoprotéines de chacune des chaînes représente 75 à 99% en poids de la masse totale du complexe et la partie oligosaccharidique qui est constituée par l'ensemble des oligosaccharides associée à chacune des chaînes représente 1 à 25% de la masse totale du complexe.

2. Complexe macromoléculaire selon la revendication 1, **caractérisé par le fait qu'**il présente une masse moléculaire supérieure à 150kDa, de préférence supérieure à 400kDa et de manière particulièrement préférée supérieure ou égale à 600kDa.

3. Complexe macromoléculaire selon l'une des revendications 1 ou 2, **caractérisé par le fait que** la lipoprotéine comprend la séquence en acides aminés SEQ ID 1.

4. Complexe macromoléculaire selon l'une des revendications 1 à 3, **caractérisé par le fait que** la séquence en acides aminés de la lipoprotéine est SEQ ID 2.

5. Complexe macromoléculaire selon l'une des revendications 1 à 4, **caractérisé par le fait que** les saccharides constituant la partie saccharidique du complexe macromoléculaire sont choisis parmi le galactose (Gal), la N-acétylgalactosamine (Gal Nac), le glucose (Glc), la N-acétylglucosamine (Glc Nac), le rhamnose (Rham), et le mannose (Man) et leurs mélanges.

6. Complexe macromoléculaire selon la revendication 5, **caractérisé par le fait que** la composition massique moyenne de galactose est comprise entre 1 et 50µg/mg de complexe macromoléculaire, de mannose est comprise entre 0,5 et 10µg/mg de complexe macromoléculaire; celle de glucose est comprise entre 3 et 80µg/mg de complexe macromoléculaire; celle de N-acétylgalactosamine est comprise entre 2 et 30µg/mg de complexe macromoléculaire; celle de N-acétylglucosamine est comprise entre 1 et 10µg/mg de complexe macromoléculaire ; celle de rhamnose est comprise entre 0,05 et 10µg/mg de complexe macromoléculaire.

7. Complexe macromoléculaire selon l'une des revendications 1 à 6, **caractérisé par le fait que** les lipides constituant la partie lipidique du complexe macromoléculaire sont choisis dans le groupe constitué des acides gras saturés longs en C14, C16, C18 et leurs mélanges.

8. Composition pharmaceutique, **caractérisée par le fait qu'**elle comprend au moins le complexe macromoléculaire selon l'une des revendications 1 à 7, à titre de principe actif, et au moins un support pharmaceutiquement acceptable.

9. Composition pharmaceutique selon la revendication 8, **caractérisée par le fait que** la concentration massique du complexe macromoléculaire selon la revendication 1 représente de 0,1 à 50µg/g de la composition pharmaceutique.

10. Composition pharmaceutique selon la revendication 8 ou 9 pour une utilisation dans le traitement ou la prévention des rhumatismes inflammatoires et de l'arthrose.

11. Composition alimentaire **caractérisée par le fait qu'**elle contient au moins ledit complexe macromoléculaire selon l'une des revendications 1 à 7, et au moins un ingrédient alimentaire.

12. Composition alimentaire selon la revendication 11, **caractérisée par le fait que** la concentration massique du complexe macromoléculaire selon la revendication 1 représente de 10µg/g à 2µg/g de la composition alimentaire.

13. Composition alimentaire selon l'une des revendications 11 ou 12, **caractérisée par le fait qu'**elle se présente sous la forme de produits alimentaires.

14. Composition nutraceutique **caractérisée par le fait qu'**elle contient au moins ledit complexe macromoléculaire selon l'une des revendications 1 à 7, et au moins un support nutraceutiquement acceptable.

15. Composition nutraceutique selon la revendication 14, **caractérisée par le fait que** la concentration massique du complexe macromoléculaire selon la revendication 1 représente de 10µg/g à 5µg/g de la composition nutraceutique.

16. Composition EP11743093.4 nutraceutique selon l'une des revendications 14 ou 15, **caractérisée par le fait qu'**elle se présente sous la forme de compléments alimentaires.

17. Composition nutraceutique selon l'une des revendications 14 à 16 pour une utilisation dans la prévention des rhumatismes inflammatoires et de l'arthrose.

18. Souche de bactéries *Bifidobacterium longum,* déposée selon le traité de Budapest sous le numéro CNCM I-3994, le 23 Mai 2008 auprès de la Collection Nationale de Cultures de Microorganismes (CNCM).

## Patentansprüche

1. Makromolekularer Komplex aus Bakterien, hergestellt von Bakterien, die dem Stamm *Bifidobacterium longum* angehören, hinterlegt gemäß dem Budapester Vertrag unter der Nummer CNCM I-3994 am 23. Mai 2008 bei der Collection Nationale de Cultures de Microorganismes (CNCM), der aus Ketten gebildet ist, die ein Lipoprotein und ein Oligosaccharid verbinden, der **dadurch gekennzeichnet ist, dass**:
- das Lipoprotein eine Molmasse von 30 kDa bis 60 kDa aufweist;
- das Oligosaccharid eine Molmasse von weniger als 15 kDa aufweist;
- der Lipoproteinbestandteil, der aus den gesamten Lipoproteinen aus jeder der Ketten besteht, 75 bis 99 Gew.% der Gesamtmasse des Komplexes darstellt und der Oligosaccharidbestandteil, der aus den gesamten Oligosacchariden, die mit jeder der Ketten verbunden sind, besteht, 1 bis 25 % der Gesamtmasse des Komplexes darstellt.

2. Makromolekularer Komplex gemäß Anspruch 1, **dadurch gekennzeichnet, dass** er eine Molmasse größer als 150 kDa, bevorzugt größer als 400 kDa und in besonders bevorzugter Weise größer als oder gleich 600 kDa aufweist.

3. Makromolekularer Komplex gemäß einem der Ansprüche 1 oder 2, **dadurch gekennzeichnet, dass** das Lipoprotein eine Aminosäuresequenz SEQ ID NO: 1 umfasst.

4. Makromolekularer Komplex gemäß einem der Ansprüche 1 bis 3, **dadurch gekennzeichnet, dass** die Aminosäuresequenz des Lipoproteins SEQ ID NO: 2 ist.

5. Makromolekularer Komplex gemäß einem der Ansprüche 1 bis 4, **dadurch gekennzeichnet, dass** die Saccharide, die den Saccharidbestandteil des makromolekularen Komplexes bilden, aus Galactose (Gal), N-Acetylgalactosamin (Gal Nac), Glucose (Glc), N-Acetylglucosamin (Glc Nac), Rhamnose (Rham) und Mannose (Man) und ihren Mischungen ausgewählt sind.

6. Makromolekularer Komplex gemäß Anspruch 5, **dadurch gekennzeichnet, dass** die durchschnittliche Massenzusammensetzung der Galactose zwischen 1 und 50 µg/mg des makromolekularen Komplexes liegt, die der Mannose zwischen 0,5 und 10 µg/mg des makromolekularen Komplexes liegt; die der Glucose zwischen 3 und 80 µg/mg des makromolekularen Komplexes liegt; die des N-Acetylgalactosamins zwischen 2 und 30 µg/mg des makromolekularen Komplexes liegt; die des N-Acetylgluocosamins zwischen 1 und 10 µg/mg des makromolekularen Komplexes liegt; die der Rhamnose zwischen 0,05 und 10 µg/mg des makromolekularen Komplexes liegt.

7. Makromolekularer Komplex gemäß einem der Ansprüche 1 bis 6, **dadurch gekennzeichnet, dass** die Lipide, die einen Lipidbestandteil des makromolekularen Komplexes bilden, aus der Gruppe ausgewählt sind, bestehend aus gesättigten Fettsäuren der Längen C14, C16, C18 und ihren Mischungen.

8. Pharmazeutische Zusammensetzung, **dadurch gekennzeichnet, dass** sie zumindest den makromolekularen Komplex gemäß einem der Ansprüche 1 bis 7 als aktiven Bestandteil und zumindest einen pharmazeutisch akzeptablen Träger umfasst.

9. Pharmazeutische Zusammensetzung gemäß Anspruch 8,
**dadurch gekennzeichnet, dass** die Massenkonzentration des makromolekularen Komplexes gemäß Anspruch 1 von 0,1 bis 50 µg/g der pharmazeutischen Zusammensetzung beträgt.

10. Pharmazeutische Zusammensetzung gemäß Anspruch 8 oder 9 zur Verwendung in der Behandlung oder der Vorbeugung von entzündlichen Rheumatismen und der Arthrose.

11. Nahrungszusammensetzung, **dadurch gekennzeichnet, dass** sie zumindest den makromolekularen Komplex gemäß einem der Ansprüche 1 bis 7 und zumindest einen Nahrungsmittelbestandteil enthält.

12. Nahrungszusammensetzung gemäß Anspruch 11, **dadurch gekennzeichnet, dass** die Massenkonzentration des makromolekularen Komplexes gemäß Anspruch 1 von 10 ng/g bis 2 µg/g der Nahrungsmittelzusammensetzung beträgt.

13. Nahrungszusammensetzung gemäß einem der Ansprüche 11 oder 12, **dadurch gekennzeichnet, dass** sie in der Form von Nahrungsmittelprodukten vorliegt.

14. Nutrazeutische Zusammensetzung, **dadurch gekennzeichnet, dass** sie zumindest den makromolekularen Komplex gemäß einem der Ansprüche 1 bis 7 und zumindest einen nutrazeutisch akzeptablen Träger enthält.

15. Nutrazeutische Zusammensetzung gemäß Anspruch 14,
**dadurch gekennzeichnet, dass** die Massenkonzentration des makromolekularen Komplexes gemäß Anspruch 1 von 10 ng/g bis 5 µg/g der nutrazeutischen Zusammensetzung beträgt.

16. Nutrazeutische Zusammensetzung gemäß einem der Ansprüche 14 oder 15, **dadurch gekennzeichnet, dass** sie in Form von Nahrungsergänzungsmitteln vorliegt.

17. Nutrazeutische Zusammensetzung gemäß einem der Ansprüche 14 bis 16 zur Verwendung in der Vorbeugung von entzündlichen Rheumatismen und der Arthrose.

18. Stamm von Bakterien *Bifidobacterium longum,* hinterlegt gemäß dem Budapester Vertrag unter der Nummer CNCM I-3994 am 23. Mai 2008 bei der Collection Nationale de Cultures de Microorganismes (CNCM).

## Claims

1. A bacterial macromolecular complex produced by bacteria belonging to the *Bifidobacterium longum* strain deposited according to the treaty of Budapest under number CNCM I-3994, on May 23, 2008, with the Collection Nationale de Cultures de Microorganismes (CNCM) [National Collection of Microorganism Cultures], consisting of chains combining a lipoprotein and an oligosaccharide, characaterized in that:
- the lipoprotein has a molecular weight of from 30 kDa to 60 kDa ;
- the oligosaccharide has a molecular weight of less than 15 kDa,
- the lipoprotein component, which consists of all the lipoproteins of each of the chains, represents from 75 to 99% by weight of the total weight of the complex, and the oligosaccharide component, which consists of all the oligosaccharides combined with each of the chains, represents from 1 to 25% of the total weight of the complex.

2. The macromolecular complex as claimed in claim 1, **characterized in that** it has a molecular weight of greater than 150 kDa, preferably greater than 400 kDa and particularly preferably greater than or equal to 600 kDa.

3. The macromolecular complex as claimed in either of claims 1 and 2, **characterized in that** the lipoprotein comprises the amino acid sequence SEQ ID 1.

4. The macromolecular complex as claimed in one of claims 1 to 3, **characterized in that** the amino acid sequence of the lipoprotein is SEQ ID 2.

5. The macromolecular complex as claimed in one of claims 1 to 4, **characterized in that** the saccharides constituting the saccharide component of the macromolecular complex are chosen from galactose (Gal), N-acetylgalactosamine (Gal Nac), glucose (Glc), N-acetylglucosamine (Glc Nac), rhamnose (Rham), and mannose (Man), and mixtures thereof.

6. The macromolecular complex as claimed in claim 5, **characterized in that** the average weight composition of galactose is between 1 and 50 µg/mg of macromolecular complex, and of mannose is between 0.5 and 10 µg/mg of macromolecular complex; that of glucose is between 3 and 80 µg/mg of macromolecular comple; that of N-acetylgalactosamine is between 2 and 30 µg/mg of macromolecular complex; that of N-acetylglucosamine is between 1 and 10 µg/mg of macromolecular complex; that of rhamnose is between 0.05 and 10 µg/mg of macromolecular complex.

7. The macromolecular complex as claimed in one of claims 1 to 6, **characterized in that** lipids constituting the lipid component of the macromolecular complex are chosen from the group consisting of long C14, C16 and C18 saturated fatty acids, and mixtures thereof.

8. A pharmaceutical composition, **characterized in that** it comprises at least the macromolecular complex as claimed in one of claims 1 to 7, as active ingredient, and at least one pharmaceutically acceptable support.

9. The pharmaceutical composition as claimed in claim 8, **characterized in that** the weight concentration of the macromolecular complex as claimed in claim 1 represents from 0.1 to 50 µg/g of the pharmaceutical composition.

10. The pharmaceutical composition as claimed in claim 8 or 9, for use in the treatment or prevention of inflammatory rheumatism and osteoarthritis.

11. A food composition, **characterized in that** it contains at least said macromolecular complex as claimed in one of claims 1 to 7, and at least one food ingredient.

12. The food composition as claimed in claim 11, **characterized in that** the weight concentration of the macromolecular complex as claimed in claim 1 represents from 10 ng/g to 2 µg/g of the food composition.

13. The food composition as claimed in either of claims 11 and 12, **characterized in that** it is in the form of food products.

14. A nutraceutical composition, **characterized in that** it contains at least said macromolecular complex as claimed in one of claims 1 to 7, and at least one nutraceutically acceptable support.

15. The nutraceutical composition as claimed in claim 14, **characterized in that** the weight concentration of the macromolecular complex as claimed in claim 1 represents from 10 ng/g to 5 µg/g of the nutraceutical composition.

16. The nutraceutical composition as claimed in either of claims 14 and 15, **characterized in that** it is in the form of food supplements.

17. The nutraceutical composition as claimed in one of claims 14 to 16, for use in the prevention of inflammatory rheumatism and osteoarthritis.

18. The strain of *Bifidobacterium longum* bacteria, deposited according to the treaty of Budapest under number CNCM I-3994, on May 23, 2008, with the Collection Nationale de Cultures de Microorganismes (CNCM) [National Collection of Microorganism Cultures].


### SEQUENCE LISTING

<110> BIFINOVE
<120> COMPLEXE MACROMOLECULAIRE D'ORIGINE BACTERIENNE ET UTILISATION DUDIT COMPLEXE MACROMOLECULAIRE POUR PREVENIR ET TRAITER LES RHUMATISMES INFLAMMATOIRES
<130> BCT110303QT
<150> FR1055432
   <151> 2010-07-05
<160> 2
<170> PatentIn version 3.5
<210> 1
   <211> 229
   <212> PRT
   <213> Bifidobacterium longum
<400> 1
   Met Thr Asn Val Arg Val Ile Lys Pro Ala Leu Ala Ala Leu Val Ala
   1               5                   10                  15
   Ala Ala Ala Cys Val Gly Gly Leu Ala Phe Ser Ser Ala Gln Pro Ala
               20                  25                  30
   Gln Ala Asp Thr Tyr Ser Asp Leu Ile Asn Ala Gln Asn Gln His Ala
           35                  40                  45
   Ala Ser Val Gln Arg Glu Ala Glu Leu Lys Gln Gln Leu Ala Gly Ala
       50                  55                  60
   Ser Gln Asp Leu Ala Asn Lys Val Leu Glu Leu Asp Asp Leu Thr Asn
   65                  70                  75                  80
   Asn Lys Ile Val Ala Ala Gln Ala Lys Val Thr Gln Ala Asn Glu Asp
                   85                  90                  95
   Ala Ala Thr Ala Gln Asp Glu Ala Asp Ala Ala Ser Gly Arg Leu Ser
               100                 105                 110
   Ala Ala Gln Lys Asp Lys Glu Thr Leu Glu Glu Gln Ile Lys Gln Thr
           115                 120                 125
   Gly Lys Asp Tyr Asp Asp Ala His Ala Ala Val Ala Gln Leu Ala Arg
       130                 135                 140
   Asp Glu Met His Gly Ser Asn Ala Ser Asp Val Met Ser Val Val Thr
   145 150 155 160
   Gly Ala Thr Ser Thr Gln Asp Phe Val Asn Ser Met Gln Ser Arg Asp
   165 170 175
   Ala Leu Ser Arg Asn Glu Ala Asn Ala Ala Ser Ser Ala Ala Thr Ser
   180 185 190
   Leu Ser Thr Ser Lys Asn Arg Gly Glu Arg Leu Ala Ala Ile Glu Lys
   195 200 205
   Gln Ile Ala Val Leu Lys Thr Gln Ala Asp Glu Lys Ala Ala Pro His
   210 215 220
   Arg Pro Pro Pro Lys
   225
<210> 2
   <211> 451
   <212> PRT
   <213> Bifidobacterium longum
<400> 2